# EUROPEAN PATENT SPECIFICATION

(11) **EP 4 181 907 B1**
(45) Date of publication and mention of the grant of the patent: **03.09.2025**
(21) Application number: 21745837.1
(22) Date of filing: 14.07.2021
(51) Int. Cl.: A61K 31/137, A61K 31/44, A61K 31/4425, A61K 31/573, A61K 38/17, A61K 45/06, A61P 25/02

(54) **TREATMENT OF CONGENITAL MYASTHENIC SYNDROME USING DOK7 GENE OR POLYPEPTIDE**
BEHANDLUNG DES KONGENITALEN MYASTHENISCHEN SYNDROMS UNTER VERWENDUNG DES DOK7-GENS ODER POLYPEPTIDS
TRAITEMENT DU SYNDROME MYASTHÉNIQUE CONGÉNITAL AU MOYEN D'UN GÈNE OU D'UN POLYPEPTIDE DOK7

(30) Priority: 15.07.2020 GB 202010894
(43) Date of publication of application: 24.05.2023
(73) Proprietor: Oxford University Innovation Limited, Oxford, OX2 0JB (GB)
(72) Inventor: BEESON, David, Oxford Oxfordshire OX3 9DS (GB)
(74) Representative: Dehns
(86) International application number: PCT/GB2021/051809
(87) International publication number: WO 2022/013554

(56) References cited:
- WO-A1-2014/167253
- US-A1- 2009 158 448
- DOWLING JAMES J ET AL: "Treating pediatric neuromuscular disorders: The future is now", AMERICAN JOURNAL OF MEDICAL GENETICS, vol. 176, no. 4, April 2018 (2018-04-01), pages 804 - 841, XP055780166
- S. ARIMURA ET AL: "DOK7 gene therapy benefits mouse models of diseases characterized by defects in the neuromuscular junction", SCIENCE, vol. 345, no. 6203, 19 September 2014 (2014-09-19), US, pages 1505 - 1508, XP055224545, ISSN: 0036-8075, DOI: 10.1126/science.1250744
- FARMAKIDIS CONSTANTINE ET AL: "Congenital Myasthenic Syndromes: a Clinical and Treatment Approach", CURRENT TREATMENT OPTIONS IN NEUROLOGY, SPRINGER US, BOSTON, vol. 20, no. 9, 21 July 2018 (2018-07-21), pages 1 - 12, XP036585714, ISSN: 1092-8480, [retrieved on 20180721], DOI: 10.1007/S11940-018-0520-7
- COSSINS JUDITH ET AL: "The spectrum of mutations that underlie the neuromuscular junction synaptopathy in DOK7 congenital myasthenic syndrome", vol. 21, no. 17, 1 June 2012 (2012-06-01), pages 3765 - 3775, XP055780044, ISSN: 0964-6906, Retrieved from the Internet <URL:https://academic.oup.com/hmg/article-pdf/21/17/3765/17255942/dds198.pdf> DOI: 10.1093/hmg/dds198

## Description

The present invention relates to a *DOK7* gene or a Dok-7 polypeptide for use in preventing or treating a congenital myasthenic syndrome (CMS) in a subject, wherein the CMS is (a) congenital myasthenic syndrome associated with AChR deficiency or (b) fast-channel congenital myasthenic syndrome (FCCMS).

Congenital myasthenic syndromes (CMSs) are a group of inherited disorders of neuromuscular transmission, characterised by fatigable muscle weakness. CMSs are due to mutations in one or more of at least thirty genes that affect presynaptic, synaptic and postsynaptic proteins in the neuromuscular junction.

The neuromuscular junction (NMJ) is the synapse between a motor neuron and skeletal muscle. It allows the motor neuron to transmit a signal to the muscle fibre, causing muscle contraction. In vertebrates, motor neurons release acetylcholine (ACh), a small molecule neurotransmitter, which diffuses across the synaptic cleft and binds to nicotinic acetylcholine receptors (nAChRs) on the cell membrane of the muscle fibre.

CMSs can arise due to mutations in acetylcholine receptor (AChR) genes; this affects the kinetics and/or expression of the AChR itself. In particular, single nucleotide substitutions, nucleotide insertions or deletions, or promoter or splicing mutations in any one of the AChR subunits, may cause loss of expression and/or function. Mutations in many other genes, such as those affecting the collagen tail subunit of acetylcholinesterase, acetyltransferase function, or the AChR-anchoring protein rapsyn can also cause a CMS.

CMSs can present themselves at different times within the life of an individual. They may arise during the fetal phase, causing fetal akinesia, or the perinatal period, during which certain conditions, such as arthrogryposis, ptosis, hypotonia, ophthalmoplegia, and feeding or breathing difficulties, may be observed. They can also activate during childhood, adolescence or adult years.

There are some existing pharmacological treatments for AChR deficiency syndromes; these are partially effective in relieving clinical symptoms.

The cholinesterase inhibitor pyridostigmine, which slows acetylcholine breakdown in the synaptic cleft, is the gold standard frontline therapy for AChR-deficient CMS patients. However, increased acetylcholine can destabilise the endplate synaptic structure, which often leads to reduced efficacy over the long term. This can be partially mitigated by co-treatment with beta2-adrenergic receptor agonists such as salbutamol (Vanhaesebrouck, A. E. *et al.,* 2019). The voltage-gated potassium channel blocker 3,4-diaminopyridine can also be used to increase ACh release from the nerve terminal and thus, like cholinesterase inhibitors, will increase the synaptic concentration of ACh.

Future options for treatments for AChR deficiency syndromes include CHRNE gene replacement therapy, as well gene-editing. However, skeletal muscle AChRs are very immunogenic, demonstrated by the prevalence of myasthenia gravis patients with autoimmune antibodies against these receptors. Therefore, gene replacement therapy to express CHRNE in patients which are deficient for the ε-subunit may cause myasthenia gravis.

Although there are symptomatic treatments available, all of patients receiving treatments are still disabled to some extent, with approximately 80% requiring some form of additional assistance with their disability which is present throughout their lives.

There is therefore a need for further treatments of AChR deficiency syndromes.

DOK7 is an effector protein which is involved in the AGRN/LRP4/MUSK/ signalling pathway; this pathway is involved in both the formation and the maintenance of the NMJ (see, for example, US 2009/0158448). Mutations in the *DOK7* gene are one major cause of CMSs. In particular, *DOK7* mutations underlie the inherited disorder familial limb-girdle myasthenia (*DOK7* myasthenia) (Beeson *et al.,* 2006).

In a mouse model of *DOK7* myasthenia, it has been shown that the administration of a human *DOK7* gene resulted in an enlargement of NMJs and substantial increases in muscle strength and life span (Arimura, A. *et al.,* 2014).

When applied to model mice of another neuromuscular disorder (i.e. autosomal dominant Emery-Dreifuss muscular dystrophy), *DOK7* gene therapy also resulted in enlargement of NMJs, as well as minor positive effects on motor activity and life span. On the basis of these results, Arimura *et al.* (2014) suggested that therapies aimed at enlarging the NMJ may be useful for a range of neuromuscular disorders.

Notably, however, Arimura *et al.* (2014) states that:
*"In contrast to many NMJ channelopathies* ..., *DOK7 myasthenia is not associated with abnormalities in the function and local density of A ChRs or the quantal release per unit size of the endplates (the region of synaptic specialization on the myotube). These observations suggest that DOK7 myasthenia should be classified* as a *synaptopathy rather than a channelopathy. Interestingly, there is accumulating evidence that NMJ structural defects may be a common feature of other neuromuscular disorders* ..., *including muscular dystrophy (MD), amyotrophic lateral sclerosis (ALS), spinal muscular atrophy (SMA), and age-related muscle weakness or sarcopenia. Indeed, studies of patients with autosomal dominant Emery-Dreifuss muscular dystrophy (AD-EDMD) and* a *mouse model of this disease have produced data suggestive of inefficient neuromuscular transmission."*

Channelopathies are diseases caused by disturbed function of ion channel subunits or the proteins that regulate them. Channelopathies affecting synaptic function are a type of synaptopathy. A synaptopathy is a disease of the brain, spinal cord or peripheral nervous system relating to the dysfunction of synapses. Arimura *et al.* (2014) is therefore suggesting here that *DOK7* myasthenia is a disorder which affects synaptic structure rather than being a disorder which is caused by disturbed function of the AChR. Notably, Arimura *et al.* (2014) do not include AChR deficiency syndromes in the above list of "other neuromuscular disorders".

Additionally, *DOK7* mutations are known to result in destabilised neuromuscular junction structures, but there is no evidence for increased breakdown and formation of synaptic structures in AChR deficiency due to CHRNE mutations.

The inventor has now found that enhancing *DOK7* expression in CHRNE-deficient mice makes them stronger and improves neuromuscular transmission. In particular, the data enclosed herein shows that treatment with *DOK7* in combination with pyridostigmine is significantly more effective than treatment with pyridostigmine alone. Therefore, for these animals, *DOK7* not only improves neuromuscular transmission in its own right, but also primes the neuromuscular junction for a greatly enhanced response to anticholinesterase medication. This combination therapy should produce an improvement in the treatment of AChR deficiency syndrome and fast-channel congenital myasthenic syndrome (FCCMS).

Both congenital myasthenic syndrome associated with AChR deficiency and FCCMS are due to loss of function mutations in nAChRs, which respectively cause a deficiency in the AChR at the motor endplate of the neuromuscular junction, or an impaired response to ACh that is abbreviated.

It is an object of the invention therefore to provide a *DOK7* gene or Dok-7 polypeptide for use in preventing or treating a congenital myasthenic syndrome (CMS) in a subject, wherein the CMS is (a) congenital myasthenic syndrome associated with AChR deficiency or (b) fast-channel congenital myasthenic syndrome (FCCMS).

The invention also provides combination therapies, wherein the *DOK7* gene or Dok-7 polypeptide is administered in combination with a cholinesterase inhibitor, a beta-adrenergic receptor agonist, an immuno-suppressor or a voltage-gated potassium channel blocker as claimed in claim 9.

Preferably, the DOK7 gene is present in the form of a rAAV-*DOK7* vector.

Preferably, the *DOK7* gene (or rAAV-DOK7 vector) or a Dok-7 polypeptide for use in preventing or treating the congenital myasthenic syndrome is used or is administered in combination with one or more of (i) a cholinesterase inhibitor, (ii) a beta-adrenergic receptor agonist, (iii) an immuno-suppressor or (iv) a voltage-gated potassium channel blocker, wherein the *DOK7 gene* or Dok-7 polypeptide and the one or more of (i)-(iv) are administered simultaneously, separately or sequentially.

A congenital myasthenic syndrome (CMS) is an inherited disorder of neuromuscular transmission, characterised by fatigable muscle weakness. CMSs are due to mutations in genes that affect presynaptic, synaptic or postsynaptic proteins in the neuromuscular junction.

In vertebrates, motor neurons release acetylcholine (ACh) which diffuses across the synaptic cleft and binds to acetylcholine receptors (AChRs) on the cell membrane of the muscle fibre.

There are two types of acetylcholine receptors: nicotinic and muscarinic. Nicotinic acetylcholine receptors (nAChR, also known as "ionotropic" acetylcholine receptors) are particularly responsive to nicotine. The nicotinic ACh receptor is an ion channel that can conduct Na⁺, K⁺ and Ca²⁺ ions, though primarily it conducts Na⁺ ions. Muscarinic acetylcholine receptors (mAChR, also known as "metabotropic" acetylcholine receptors) are particularly responsive to muscarine. Nicotinic acetylcholine receptors respond to the neurotransmitter acetylcholine; these are present in neuromuscular junctions. In the context of this invention, the CMS is one which is associated with (i.e. due to or caused by) one or more mutations in a nicotinic acetylcholine receptor.

Nicotinic AChRs are made up of five subunits, arranged symmetrically around a central pore. Each subunit comprises four transmembrane domains, with both the N- and C-termini located extracellularly.

In vertebrates, nicotinic receptors are broadly classified into two subtypes based on their primary sites of expression: muscle-type nicotinic receptors and neuronal-type nicotinic receptors.

Muscle-type receptors are concentrated at the neuromuscular junction. They are either the embryonic form, composed of α1, β1, γ, and δ subunits in a 2:1:1:1 ratio; or the adult form composed of α1, β1, δ, and ε subunits in a 2:1:1:1 ratio.

In the context of this invention, the CMS is one which is associated with (i.e. due to or caused by) one or more mutations in a muscle-type nicotinic acetylcholine receptor.

Mutations in nAChRs can give rise to a number of different deficiencies and syndromes including AChR deficiency, Fast channel congenital myasthenic syndrome (FCCMS), Low conductance syndrome and Slow channel syndrome ("A nomenclature and classification for the congenital myasthenic syndromes: preparing for FAIR data in the genomic era", Thompson R, Abicht A, Beeson D, Engel AG, Eymard B, Maxime E, Lochmüller H. Orphanet J Rare Dis. 2018 Nov 26; 13(1):211).

Congenital myasthenic syndrome associated with AChR deficiency is a disorder of the postsynaptic neuromuscular junction (NMJ) clinically characterized by early-onset muscle weakness with variable severity. (AChR deficiency: OMIM #608931: Myasthenic syndrome, congenital, 4c, associated with acetylcholine receptor deficiency; CMS4c (CHRNE); OMIM 616323: Myasthenic syndrome, congenital, 3c, associated with acetylcholine receptor deficiency; CMS3c (CHRND); OMIM 616314: Myasthenic syndrome, congenital, 2c, associated with acetylcholine receptor deficiency; CMS2c (CHRNB); Congenital myasthenic syndrome due to primary acetylcholine receptor deficiency caused by pathogenic variants in *CHRNA1* occurs but does not have an OMIM #.) Electrophysiologic studies show low amplitude of the miniature endplate potential (MEPP) and current (MEPC) resulting from deficiency of AChR at the endplate. Patients with mutations in the CHRNE gene maintain low level expression of the fetal subunit CHRNG (100730). CHRNE mutations are the most common cause of AChR deficiency, but all forms of AChR deficiency may respond to treatment with cholinergic agents, pyridostigmine, or amifampridine.

Fast-channel congenital myasthenic syndrome (FCCMS) is a disorder of the postsynaptic neuromuscular junction (NMJ) characterized by early-onset progressive muscle weakness. (OMIM 608930: Myasthenic syndrome, congenital, 1b, fast-channel; CMS1b (CHRNA1); OMIM 616322: Myasthenic syndrome, congenital, 3b, fast-channel; CMS3b (CHRND); OMIM: 616324: Myasthenic syndrome, congenital, 4b, fast-channel; CMS4b (CHRNE); Fast-channel congenital myasthenic syndrome due to an acetylcholine receptor defect caused by pathogenic variants in *CHRNB1* occurs but does not have OMIM#.) The disorder results from kinetic abnormalities of the acetylcholine receptor (AChR) channel, specifically from abnormally brief opening and activity of the channel, with a rapid decay in endplate current and a failure to reach the threshold for depolarization. Treatment with pyridostigmine or amifampridine may be helpful.

Congenital myasthenic syndromes associated with AChR deficiency and FCCMS are both due to loss of function mutations in nAChRs, which cause, respectively, a deficiency in the AChR at the motor endplate of the neuromuscular junction or attenuated ion channel openings.

The invention would not be beneficial for the treatment of Slow channel syndrome because this syndrome relates to a gain of function of the AChR.

The human genes encoding the muscle-type nAChRs are the following:

| **Subunit** | **Gene** | **cDNA sequence** | **Polypeptide sequence** |
|---|---|---|---|
| α1 | CHRNA1 | SEQ ID NO: 1 | SEQ ID NO: 2 |
| β1 | CHRNB1 | SEQ ID NO: 3 | SEQ ID NO: 4 |
| δ | CHRND | SEQ ID NO: 5 | SEQ ID NO: 6 |
| ε | CHRNE | SEQ ID NO: 7 | SEQ ID NO: 8 |
| γ | CHRNG | SEQ ID NO: 9 | SEQ ID NO: 10 |

In some particularly-preferred embodiments, the one or more mutations are in the CHRNE gene.

The mutations may be one or more substitutions, insertions or deletions of one or more nucleotides in the muscle-type nAChR genes. The mutations may also be ones which affect RNA splicing of the muscle-type nAChR genes.

Preferably, at least one of the mutations is a non-synonymous mutation, i.e. one which changes the amino acid sequence of the corresponding polypeptide. Some synonymous mutations can affect splicing and thus are pathogenic.

In some embodiments, the one or more mutations is a single nucleotide substitution or single nucleotide deletion.

The one or more mutations may be homozygous or heterozygous mutations, or a mixture of both. The one or more mutations may also be compound heterozygotes, i.e. with heteroallelic mutations. Preferably, the one or more mutations are homozygous mutations.

Null mutations of the adult AChR ε-subunit gene are the most common cause of AChR deficiency syndrome. Preferably, therefore, the mutation is a null mutation in the CHRNE gene.

Preferably, one or more of the mutations are recessive mutations, causing loss of function for the AChR at the neuromuscular junction.

In some preferred embodiments, the CMS disorder is due to one or more of the following:
(i) a mutation in the CHRNA1 gene which leads to AChR deficiency,
(ii) a mutation in the CHRNB1 gene which leads to AChR deficiency,
(iii) a mutation in the CHRND gene which leads to AChR deficiency,
(iv) a mutation in the CHRNE gene which leads to AChR deficiency,
(v) a mutation in the CHRNG gene which leads to AChR deficiency,
(vi) a mutation in the CHRNE gene which leads to FCCMS,
(vii) a mutation in the CHRNA1 gene which leads to FCCMS,
(viii) a mutation in the CHRNB1 gene which leads to FCCMS, or
(ix) a mutation in the CHRND gene which leads to FCCMS.

Preferably, the CMS disorder is due to a mutation in the CHRNE gene which leads to AChR deficiency.

Numerous mutations have been identified in the AChR gene as being associated with AChR deficiency or FCCMS. Reference may be made this regard to Engel AG, et al. "Congenital myasthenic syndromes: pathogenesis, diagnosis, and treatment," Lancet Neurology (2015), vol. 14, issue 4, pp. 420-434. Further details of mutations in the CHRNE gene may be found at www.ncbi.nlm.nih.gov/clinvar/?term=CHRNE[gene].

In some embodiments, the mutation is selected from the following:

| **Gene** | **Nucleotide mutation** | **Polypeptide mutation** | **CMS disorder** |
|---|---|---|---|
| CHRNE | c.1181_1187dup | p.Glu387fs | AChR deficiency |
| CHRNE | c.1327delG | p.Glu443fs | AChR deficiency |
| CHRNE | c.569insA | p.Ile189fs | AChR deficiency |
| CHRNE | c.1429delG | p.Gly476fs | AChR deficiency |
| CHRNE | c.840_842del | p.Val311del | AChR deficiency |
| CHRNE | c.372C>A | p.Tyr124* | AChR deficiency |
| CHRNE | c.687ins2 | p.Asp229fs | AChR deficiency |
| CHRNE | c.293T>C | p. Leu98Pro | AChR deficiency |
| CHRNE | c.1112C>T | p.Pro351Leu | AChR deficiency |
| CHRNE | c.27_32del6 | p.9_10delLL | AChR deficiency |
| CHRNE | c.317G>A | p.Arg106Lys | AChR deficiency |
| CHRNE | c.662+2T>A | Splice site | AChR deficiency |
| CHRNE | c.1351G>G | p.Ala431Pro | AChR deficiency |
| CHRNE | c.103T>C | p.Tyr35His | AChR deficiency |
| CHRNE | c.130insG | p.Arg43fs | AChR deficiency |
| CHRNE | c.1093insT | p.Arg364fs | AChR deficiency |
| CHRNE | c.1266ins19 | p.422fs | AChR deficiency |
| CHRNE | c.614_620del | p.Trp205fs | AChR deficiency |
| CHRNE | c.529_531delGAG | p.Glu177del | AChR deficiency |
| CHRNE | c.468C>T | p.Ser163Leu | AChR deficiency |
| CHRNE | c.971delT | p.Val303fs | AChR deficiency |
| CHRNE | c.-156C>T | Promoter | AChR deficiency |
| CHRNE | c.905C>G | p.Pro302Arg | AChR deficiency |
| CHRNE | c.501-2A>C | Splicing | AChR deficiency |
| CHRNE | c.803-2A>G | Splicing | AChR deficiency |
| CHRNE | c.94delins37 | p.Ser33LeuArg* | AChR deficiency |
| | c. IVS8-1 G>A | p.306fs | AChR deficiency |
| | c.918-1G>A | | |
| CHRNE | c.422C>T | p.Pro141Leu | Fast channel |
| CHRNA1 | c.934C>T | p.Gln312* | AChR deficiency |
| CHRNA1 | c.257G>A | p.Arg86His | AChR deficiency |
| CHRNA1 | c.1253A>G | p.Glu418Gly | AChR deficiency |
| CHRNA1 | c.224G>A | p.Arg75His | AChR deficiency |
| CHRNA1 | c.1321G>A | p.Gly441Arg | AChR deficiency |
| CHRNB1 | c.621C>T | p.Arg241 cys | AChR deficiency |
| CHRNB1 | c.345C>T | p.Cys149Arg | AChR deficiency |
| CHRND | c.226A>G | p.Asn76Asp | AChR deficiency |
| CHRND | c.1007G>A | p.Arg336Gln | AChR deficiency |
| CHRND | c.870-42_-25del | Splicing | AChR deficiency |
| CHRND | C.749C>A | p.Pro250Gln | AChR deficiency |

Any of CHRNA1, CHRNB1, CHRND and CHRNE can have mutations causing a FCCMS.

The subject is preferably a mammalian subject. The mammal may be human or non-human. For example, the subject may be a farm mammal (e.g. sheep, horse, pig, cow or goat), a companion mammal (e.g. cat, dog or rabbit) or a laboratory test mammal (e.g. mouse, rat or monkey).

Preferably, the subject is a human. The subject may be male or female. The human may, for example, be 0-10, 10-20, 20-30, 30-40, 40-50, 50-60, 60-70, 70-80, 80-90, 90-100 or above 100 years old.

Dok-7 is a non-catalytic cytoplasmic adaptor protein that is expressed specifically in muscle and is essential for the formation of neuromuscular synapses. Dok-7 is an activator of the receptor kinase MuSK. The properties of Dok-7 are described in US 2009/0158448. Dok-7 contains pleckstrin homology (PH) and phosphotyrosine-binding (PTB) domains which are important for Dok-7 function.

The nucleotide and amino acid sequences of the human *DOK7* gene and Dok-7 polypeptide are given herein as SEQ ID NOs: 11 and 12, respectively. As used herein, the term *"DOK7* gene" includes, but is not limited to:
(a) a polynucleotide molecule whose nucleotide sequence comprises or consists of the nucleotide sequence given in SEQ ID NO: 11;
(b) a polynucleotide molecule whose nucleotide sequence comprises or consists of a variant of the nucleotide sequence given in SEQ ID NO: 11, the variant having at least 90%, 95% or 99% sequence identity to SEQ ID NO: 11; and
(c) a polynucleotide molecule whose nucleotide sequence comprises or consists of a nucleotide sequence which encodes:
   (i) a polypeptide whose amino acid sequence is given in SEQ ID NO: 12, or
   (ii) a variant of (i), the variant having at least 90%, 95% or 99% sequence identity to SEQ ID NO: 12,
   and wherein the variant is or encodes a non-catalytic cytoplasmic adaptor protein.

As used herein, the term "Dok-7 polypeptide" preferably refers to a polypeptide whose amino acid sequence comprises or consists of the amino sequence as given in SEQ ID NO: 12, or variant thereof having at least 90%, 95% or 99% sequence identity thereto and wherein the variant is a non-catalytic cytoplasmic adaptor protein.

Numerous variants of *DOK7* are known (e.g. Cossins J, Liu WW, Belaya K, Maxwell S, Oldridge M, Lester T, Robb S, Beeson D. The spectrum of mutations that underlie the neuromuscular junction synaptopathy in DOK7 congenital myasthenic syndrome. Hum Mol Genet. 2012; 21:3765-3775). The invention extends to all non-pathogenic variants of *DOK7.*

There are many established algorithms available to align two amino acid or nucleic acid sequences. Typically, one sequence acts as a reference sequence, to which test sequences may be compared. The sequence comparison algorithm calculates the percentage sequence identity for the test sequence(s) relative to the reference sequence, based on the designated program parameters. Alignment of amino acid or nucleic acid sequences for comparison may be conducted, for example, by computer-implemented algorithms (e.g. GAP, BESTFIT, FASTA or TFASTA), or BLAST and BLAST 2.0 algorithms.

Percentage amino acid sequence identities and nucleotide sequence identities may be obtained using the BLAST methods of alignment (Altschul et al. (1997), "Gapped BLAST and PSI-BLAST: a new generation of protein database search programs", Nucleic Acids Res. 25:3389-3402; and http://www.ncbi.nlm.nih.gov/BLAST). Preferably the standard or default alignment parameters are used.

Standard protein-protein BLAST (blastp) may be used for finding similar sequences in protein databases. Like other BLAST programs, blastp is designed to find local regions of similarity. When sequence similarity spans the whole sequence, blastp will also report a global alignment, which is the preferred result for protein identification purposes. Preferably the standard or default alignment parameters are used. In some instances, the "low complexity filter" may be taken off.

BLAST protein searches may also be performed with the BLASTX program, score=50, wordlength=3. To obtain gapped alignments for comparison purposes, Gapped BLAST (in BLAST 2.0) can be utilized as described in Altschul et al. (1997) Nucleic Acids Res. 25: 3389. Alternatively, PSI-BLAST (in BLAST 2.0) can be used to perform an iterated search that detects distant relationships between molecules. (See Altschul et al. (1997) supra). When utilizing BLAST, Gapped BLAST, or PSI-BLAST, the default parameters of the respective programs may be used.

With regard to nucleotide sequence comparisons, MEGABLAST, discontiguous-megablast, and blastn may be used to accomplish this goal. Preferably the standard or default alignment parameters are used. MEGABLAST is specifically designed to efficiently find long alignments between very similar sequences. Discontiguous MEGABLAST may be used to find nucleotide sequences which are similar, but not identical, to the nucleic acids of the invention.

The BLAST nucleotide algorithm finds similar sequences by breaking the query into short subsequences called words. The program identifies the exact matches to the query words first (word hits). The BLAST program then extends these word hits in multiple steps to generate the final gapped alignments. In some embodiments, the BLAST nucleotide searches can be performed with the BLASTN program, score=100, wordlength=12.

One of the important parameters governing the sensitivity of BLAST searches is the word size. The most important reason that blastn is more sensitive than MEGABLAST is that it uses a shorter default word size (11). Because of this, blastn is better than MEGABLAST at finding alignments to related nucleotide sequences from other organisms. The word size is adjustable in blastn and can be reduced from the default value to a minimum of 7 to increase search sensitivity.

A more sensitive search can be achieved by using the newly-introduced discontiguous megablast page (www.ncbi.nlm.nih.gov/Web/Newsltr/FallWinter02/blastlab.html). This page uses an algorithm which is similar to that reported by Ma et al. (Bioinformatics. 2002 Mar; 18(3): 440-5). Rather than requiring exact word matches as seeds for alignment extension, discontiguous megablast uses a non-contiguous word within a longer window of template. In coding mode, the third base wobbling is taken into consideration by focusing on finding matches at the first and second codon positions while ignoring the mismatches in the third position. Searching in discontiguous MEGABLAST using the same word size is more sensitive and efficient than standard blastn using the same word size. Parameters unique for discontiguous megablast are: word size: 11 or 12; template: 16, 18, or 21; template type: coding (0), non-coding (1), or both (2).

In some embodiments, the BLASTP 2.5.0+ algorithm may be used (such as that available from the NCBI) using the default parameters.

In other embodiments, a BLAST Global Alignment program may be used (such as that available from the NCBI) using a Needleman-Wunsch alignment of two protein sequences with the gap costs: Existence 11 and Extension 1.

As used here, the term "preventing" includes prophylactic use of a *DOK7* gene or a Dok-7 polypeptide in order to prevent, hinder or slow the progression of the CMS. As used here, the term "treating" includes alleviating the symptoms of the CMS.

Adeno-associated viruses (AAV) are small viruses that infect humans and some other primate species. They belong to the genus *Dependoparvovirus,* which in turn belongs to the family *Parvoviridae.* They are small (20 nm) replication-defective, non-enveloped viruses.

Wild-type AAV has attracted considerable interest from gene therapy researchers due to a number of features. Chief amongst these is the virus's apparent lack of pathogenicity. It can also infect non-dividing cells and has the ability to stably integrate into the host cell genome at a specific site (designated AAVS1) in the human chromosome 19.

Development of recombinant AAVs (rAAVs) as gene therapy vectors, however, has eliminated this integrative capacity by removal of the *rep* and *cap* genes from the DNA of the vector. The desired transgene together with a promoter to drive transcription of the transgene is inserted between the inverted terminal repeats (ITRs) that aid in concatemer formation in the nucleus after the single-stranded vector DNA is converted by host cell DNA polymerase complexes into double-stranded DNA. rAAV-based gene therapy vectors form episomal concatemers in the host cell nucleus. In non-dividing cells, these concatemers remain intact for the life of the host cell. In dividing cells, rAAV DNA is lost through cell division, since the episomal DNA is not replicated along with the host cell DNA.

The use of rAAV vectors in gene therapy is well known in the art. In particular, rAAV vectors have been used to deliver therapeutic genes to skeletal muscle and other tissues (e.g. M. Nonnenmacher, T. Weber, Gene Ther. 19, 649-658 (2012); and F. Mingozzi, K. A. High, Nat. Rev. Genet. 12, 341-355 (2011)). More specifically, the production and use of rAAV-DOK7 vectors is described in Arimura *et al.* (2014).

In some preferred embodiments, the *DOK7* gene is present in and is used in a rAAV vector (i.e. rAAV-DOK7). In this case, the rAAV vector comprises AAV inverted terminal repeats, which flank a promoter which is operably-associated with a *DOK7* gene. AAV *rep* and *cap* genes are both preferably absent.

Preferably, the promoter is CMV, the human muscle creatine kinase promoter MHCK7, the human skeletal actin (HSA), the creatine kinase 8 (CK8) or the desmin (Des) promoter. In other preferred embodiments, the promoter is a NMJ-specific promoter, e.g. an AChR gene promoter, such as the human CHRNAB1 promoter region (Webster *et al.* (2013)).

The rAAV may be any suitable serotype. Preferably, the rAAV is AAV9.

The *DOK7* gene or Dok-7 polypeptide may be administered to the subject in a suitable pharmaceutically-acceptable composition, optionally together with one or more pharmaceutically-acceptable diluents, excipients and/or carriers. For example, the pharmaceutically-acceptable composition may comprise PBS, preferably with Pluronic (e.g. 0.001%).

The composition may comprise a suitable number of viral genomes (vg). The dose will depend on the subject size, the potential immune response to the AAV and the route of administration. Preferably, one dose comprises 1 x 10¹⁰ to 5 x 10¹⁴ viral genomes per kilogram subject.

The AAV may be administered by any suitable route, including intra-venous (IV), intra-thecal (IT) and intra-peritoneal (IP) injection. Preferably, the AAV are administered by intravenous injection. Preferably, only a single dose is given to the subject.

In some preferred embodiments, the *DOK7* gene or Dok-7 polypeptide for use in preventing or treating the congenital myasthenic syndrome may be used or administered to the subject in combination with one or more of the following:
(i) a cholinesterase inhibitor,
(ii) a beta-adrenergic receptor agonist,
(iii) an immuno-suppressor, and
(iv) a voltage-gated potassium channel blocker,
wherein the *DOK7* gene or Dok-7 polypeptide and the one or more of (i)-(iv) are administered to the subject simultaneously, separately or sequentially.

During nerve transmission, acetylcholine (Ach) diffuses across the synaptic cleft and binds to receptors on the post-synaptic membrane, causing an influx of Na+, resulting in depolarization. If large enough, this depolarization results in an action potential. To prevent constant stimulation once the ACh is released, an enzyme called acetylcholinesterase is present in the endplate membrane close to the receptors on the post synaptic membrane, and quickly hydrolyses ACh. ACh inhibitors inhibit acetylcholinesterase in the synaptic cleft, thus slowing down the hydrolysis of acetylcholine. Examples of acetylcholinesterase inhibitors include neostigmine bromide (Prostigmin), pyridostigmine bromide (Mestinon) and ambenonium chloride (Mytelase). Preferably, the cholinesterase inhibitor is pyridostigmine.

Pyridostigmine is a quaternary carbamate inhibitor of cholinesterase that does not cross the blood-brain barrier; it carbamylates about 30% of peripheral cholinesterase enzyme. The carbamylated enzyme eventually regenerates by natural hydrolysis and excess ACh levels revert to normal. Pyridostigmine has previously been used to treat muscle weakness in people with myasthenia gravis and some forms of congenital myasthenic syndrome.

Appropriate doses of the acetylcholinesterase inhibitors may readily be determined by those of skill in the art, taking into account the subject.

Pyridostigmine may be used at between 4mg/kg/day and 8mg/kg/day. Higher doses of up to 10mg/kg/day may be used in some circumstances, but this can be toxic/detrimental to the NMJ and have marked side effects. The dose will depend on how quickly the medicament is metabolised out of the system, which varies according to the subject, but it is usually given to humans every 3 to 4 hours. Pyridostigmine is readily available in 60mg or 120 mg tablets, or can be made up as a suspension. In some cases, it is given 3, 4 or 5 times per day depending on subject, to obtain a dose of about 6 mg/kg/day. Neostigmine may be used at a concentration of 15-375 mg/day for adults. Mytelase may be used at 20-200mg per day for adults.

The use of acetylcholinesterase inhibitors can destabilise the endplate synaptic structure, which often leads to reduced efficacy over the long term. This can be partially mitigated by co-treatment with beta-adrenergic receptor agonists. Beta-adrenergic receptor agonists have previously been used for the treatment of congenital myasthenia (e.g. Rodriguez Cruz *et al.,* 2015; and Vanhaesebrouck, A. E., *et al.,* 2019). Suitable beta-adrenergic receptor agonists include Salbutamol (albuterol), Terbutaline, Levalbuterol, Pirbuterol and Ephedrine. Preferably, the beta-adrenergic receptor agonist is a beta2-adrenergic receptor agonist, e.g. Ephedrine or Salbutamol.

Most preferably, the beta2-adrenergic receptor agonist is salbutamol. Appropriate doses of the beta-adrenergic receptor agonist may readily be determined by those of skill in the art, taking into account the subject. Suitable doses of salbutamol include 8-16 mg twice per day for adults, and 2-4 mg twice per day for children (depending on weight). Suitable doses of Ephedrine include 45 mg twice per in adults, and about 30 mg/day in children (depending on weight).

In order to reduce an immune response to the rAAV, an immuno-suppressor may also be administered to the subject. Examples of suitable immuno-suppressors include prednisone, dexamethasone, methylprednisolone, rapamycin and Rituximab. The immuno-suppressor may also be an agent or device to reduce pathogenic immunoglobulin (IgG). An example of an agent to reduce pathogenic IgG is Imlifidase. Preferably, the immuno-suppressor is prednisone, preferably administered at 60 mg/day.

A voltage-gated potassium channel blocker may also be administered to the subject.

Examples of voltage-gated potassium channel blockers include 3,4-diaminopyridine (3,4-DAP) and amifampridine (3,4-DAPP).

In some embodiments, the *DOK7* gene or Dok-7 polypeptide for use in preventing or treating the congenital myasthenic syndrome (CMS) may be used or administered in combination with one or more of the following:
(i) a cholinesterase inhibitor,
(ii) a beta-adrenergic receptor agonist,
(iii) an immuno-suppressor,
(iv) a voltage-gated potassium channel blocker,
wherein the DOK7 gene or Dok-7 polypeptide and the one or more of (i)-(iv) are administered simultaneously, separately or sequentially.

The *DOK7* gene/Dok-7 polypeptide and each of (i)-(iv) may be administered by the same or different routes.

The invention also provides a pharmaceutical composition comprising a *DOK7* gene or Dok-7 polypeptide in combination with one or more of (i)-(iv) above, wherein the pharmaceutical combination is in the form of a combined preparation for simultaneous, separate or sequential use and wherein the pharmaceutical combination is for use in the treatment of the specified CMS ((a) or (b)).

### BRIEF DESCRIPTION OF THE FIGURES

Figure 1. rAAV-*DOK7* treated GAMR mice (low dose).
Figure 2. rAAV-*DOK7* GAMR mice (low dose) hang test (males, n=10).
Figure 3. 30 Hz stimulation train in GAMR mice.
Figure 4. rAAV-*DOK7* treated GAMR mice (low dose), pyridostygmine trial (males, n=4).

### EXAMPLES

The present invention is further illustrated by the following Examples, in which parts and percentages are by weight and degrees are Celsius, unless otherwise stated. It should be understood that these Examples, while indicating preferred embodiments of the invention, are given by way of illustration only. Thus, various modifications of the invention in addition to those shown and described herein will be apparent to those skilled in the art from the foregoing description.

### Example 1: Proof of concept

Initial proof of concept studies using a modest dose of rAAV-DOK7 (4.5 x 10^11 viral genomes) in combination with pyridostigmine were carried out in the well-established mouse model of CHRNE deficiency (Cossins *et al.,* 2004).

An AAV serotype 9 plasmid from Vigene (pAv-C-EGFP AAV9) was used that contains a reporter EGFP, a CMV promoter and the inverted terminal repeats for the AAV. DOK7 cDNA, the derivation of which is described in Cossins *et al.* (Cossins J, Liu WW, Belaya K, Maxwell S, Oldridge M, Lester T, Robb S, Beeson D. The spectrum of mutations that underlie the neuromuscular junction synaptopathy in DOK7 congenital myasthenic syndrome. Hum Mol Genet. 2012 ;21:3765-3775), was amplified with primers containing restriction sites for AsisI on the forward and Xhol on the reverse primer, with the restriction site Xhol to come exactly in the place of the stop codon of DOK7 cDNA so the reading frame runs into EGFP and thus to produce a tagged Dok-7.

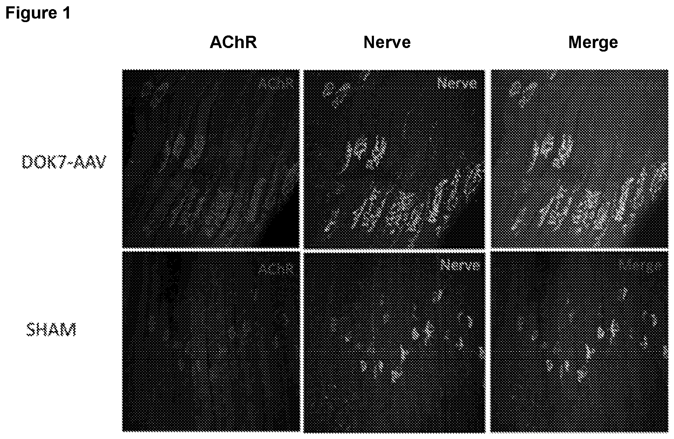

As shown in Figure 1, motor endplates from the diaphragm of AChR deficiency model mice (GAMR) after a single intraperitoneal (IP) injection at 3 weeks of age with 4.5x 10¹¹ viral genomes of rAAV-*DOK7* or sham (rAAV without *DOK7*) showed enlarged neuromuscular junctions induced by the rAAV-*DOK7*. AChR are labelled with α-bungarotoxin (red) and nerve terminals with synaptophysin (green).

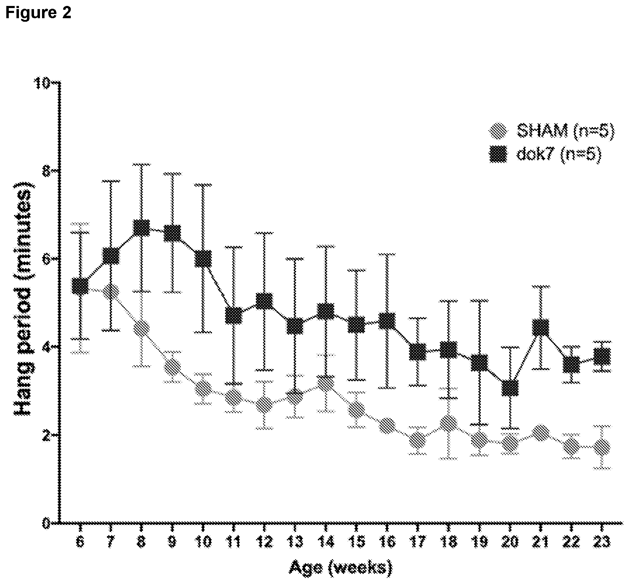

As shown in Figure 2, rAAV-*DOK7* treatment enhanced the hang-period for the AChR deficiency mice. Mice were given a single IP injection at 3 weeks of age with either rAAV-*DOK7* (n=5) or sham injection with rAAV not harbouring DOK7 cDNA (n=5). Mice were randomly allocated to the 'sham' or 'dok7' groups, and the injections were conducted with the technician/student blinded as to which treatment was injected. The inverted screen test involves placing the mouse on a wire mesh/screen, inverting the mesh and then timing the period for which the mouse can hang on before fatigue of muscles causes them to fall onto a soft bed. We have found this to be an excellent technique for monitoring myasthenic weakness (Cossins *et al.,* 2004; Vanhaesebrouck *et al.,* 2019).

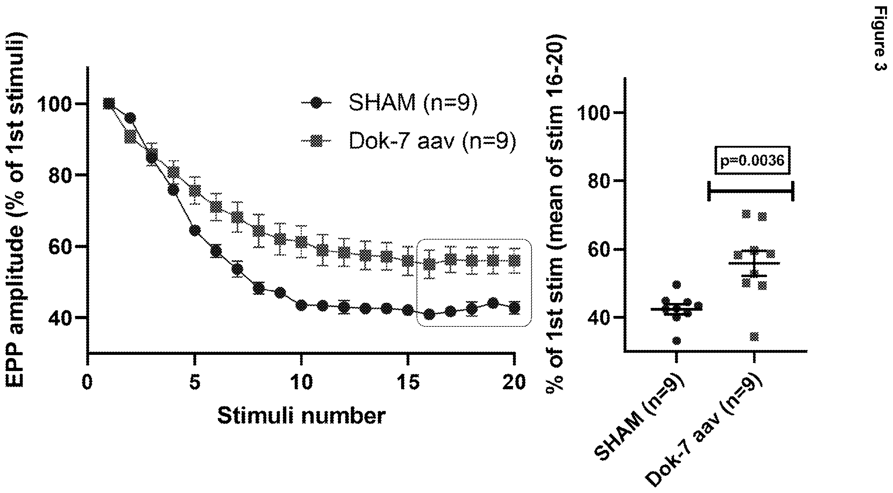

As shown in Figure 3, rAAV-*DOK7* treated mice show a less marked reduction of endplate potential amplitude following repetitive stimulation. Hemi-diaphragm preparations were made from the mice, sodium channels blocked, and the phrenic nerve stimulated by a chain of stimuli at 30 Hz, and the endplate potentials recorded (as described in Vanhaesebrouck *et al.,* 2019). The reduction in endplate potentials due to the train of stimuli were recorded for each mouse and the endplate potentials at the 16-20th stimuli compared to the initial stimulus.

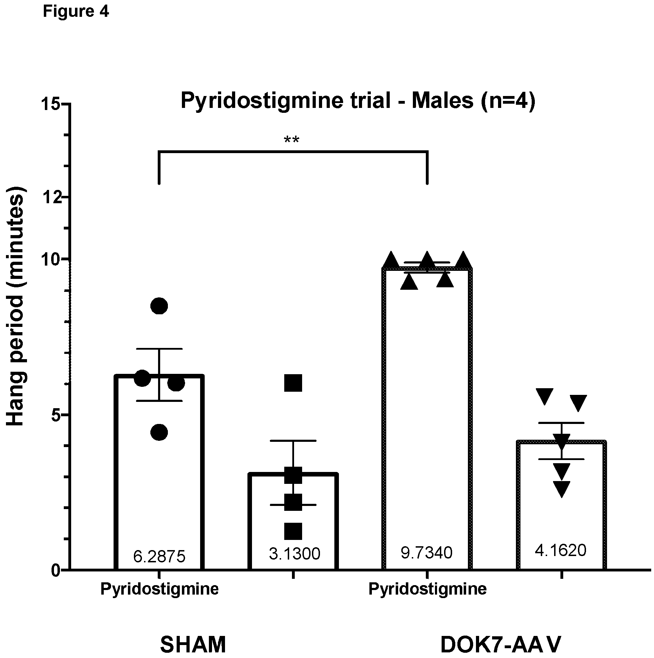

Figure 4 shows an inverted-screen test for the response to pyridostigmine on rAAV-*DOK7* treated or sham-injected AChR deficiency mice (as reported in Figure 1). At around 16 weeks of age, pyridostigmine at a level of 14 mg/kg/day ingestion was included in the water. After 24 hours, the time for the inverted screen was recorded and compared to the time immediately prior to adding pyridostigmine (details of administration of pyridostigmine are given in Vanhaesebrouck *et al.,* 2019).

These results show that rAAV-*DOK7* treatment both improved mouse strength and also markedly enhanced the response of the model to treatments using cholinesterase inhibitors (approximately 4-fold).

### Example 2: Delivery optimisation and biodistribution assessment

To determine the method of delivery that yields the highest rAAV-*DOK7* biodistribution and transduction efficiency, 3 different methods of injection are tested: intra-venous (IV, into the mouse tail vein), intra-thecal (IT) and intra-peritoneal (IP) injection. Wild-type mice are used for this experiment. Four mice are injected with 5x 10^11 viral genomes at 3 weeks of age using each method, and four uninjected mice are included as negative controls. Mice are euthanized 2 weeks after injection, and their extensor digitorum longus (EDL), soleus, and diaphragm muscles are harvested and examined post-mortem.

To ascertain transduction efficiency, qPCR is carried out on genomic DNA extracted from each muscle harvested to quantify the number of viral genomes (Vg) present per mouse diploid genome (Dg). Fluorescent microscopy is used to determine the size of the neuromuscular junctions in the harvested muscles of each animal. The primary measure of success is the highest mean Vg/Dg ratio between the 3 muscles of each treatment group. The delivery method that gives the highest transduction efficiency across the different muscles is used in all future experiments in this project.

### Example 3: Toxicology/immune response

To examine any toxic effects and immune responses to the rAAV-*DOK7*/pyridostigmine treatment, homozygous CHRNE deficient mice are injected with the highest dose of treatment (1.5x 10^12 viral genomes of rAAV-*DOK7*, 14mg/kg/day pyridostigmine) that is used in this trial with or without an immune suppressor, or a sham treatment (buffer). Four mice are included in each treatment group; they are observed and weighed every day for 2 weeks, before they are euthanized. A CRO is sub-contracted to conduct toxicology and immune response assessments. A scientist from the CRO is present when the mice are euthanized to collect the necessary samples from each animal. If only small effects are observed with or without administration of the immune suppressor, then future treatments are administered without any immune suppression. If a severe immune response is observed without an immune suppressor, but not with the immune suppressor, then future treatments are administered with the immune suppressor. If high levels of toxicity or immune response are observed with or without an immune suppressor, then the experiment is repeated at a lower dose of treatment.

rAAV9 is used, which has a proven track record for clinical safety; and no toxic effects have been observed in preliminary experiments, or published studies using the *DOK7-*CMV-AAV9 construct which is tested. Therefore, severe toxicity is not anticipated.

### Example 4: Dose/response assessment

rAAV-*DOK7* is injected into homozygous CHRNE deficient mice using the most efficient method determined in the Example 2, at a maximum dose determined by Example 3; pyridostigmine is added to the drinking water at 14mg/kg/day. Two other doses of rAAV-*DOK7,* 3x and 10x lower than the maximum, are also tested to determine the dose/response relationship of the treatment. Mice injected with the negative control sham treatment, fed with pyridostigmine plus salbutamol treatment (14mg/kg/day and 45mg/kg/day, respectively), and untreated wild-type litter mates are also tested in parallel.

The effectiveness of the treatments is assessed by weekly inverted-hang tests, and monthly neurophysiology examinations over 12 months. Toxicology and immune activation tests are carried out after the animals are euthanized; and EDL, soleus and diaphragm muscles are harvested to assess super-synapse formation using fluorescent microscopy, as well as transduction efficiency using qPCR. Hemi-diaphragm preparations are made for detailed electrophysiological experiments to examine the effects of treatment on neuromuscular transmission.

The inverted-hang test is the primary outcome measure of treatment efficacy. Significant improvement in efficacy of the rAAV-*DOK7*/pyridostigmine combination treatment over the pyridostigmine/salbutamol treatment, without significant toxic effects or immune activation, is expected. The dose/response assessment determines the minimum dose that gives the maximum efficacy. Longitudinal data acquired over 12 months gives an indication of the long term effects of the treatment. Post-mortem fluorescent microscopy, qPCR, and electrophysiology data assesses the long term stability of the viral genomes, and the super-synapses induced.

### REFERENCES

Arimura, S., et al. (2014). Science 345(6203): 1505-1508.
Beeson D, Higuchi O, Palace J, Cossins J, Spearman H, Maxwell S, Newsom-Davis J, Burke G, Fawcett P, Motomura M, Muller J, Lochmuller H, Slater C, Vincent A, Yamanashi Y. Dok-7 mutations underlie a neuromuscular junction synaptopathy. Science 2006; 313:1975-1978.
Cossins, J., R. et al. (2004). Hum Mol Genet 13(23): 2947-2957.
Cossins J, Liu WW, Belaya K, Maxwell S, Oldridge M, Lester T, Robb S, Beeson D. The spectrum of mutations that underlie the neuromuscular junction synaptopathy in DOK7 congenital myasthenic syndrome. Hum Mol Genet. (2012); 21:3765-3775).
Engel AG, Shen XM, Selcen D, Sine SM Congenital myasthenic syndromes: pathogenesis, diagnosis, and treatment. Lancet Neurol. 2015 Apr;14(4):420-34. doi: 10.1016/S1474-4422(14)70201-7.PMID: 25792100
Inoue, A., et al. (2009). Sci Signal 2(59): ra7.
Rodriguez Cruz, P. M., et al. (2015). Neurology 85(12): 1043-1047.
Thompson R, Abicht A, Beeson D, Engel AG, Eymard B, Maxime E, Lochmüller H. A nomenclature and classification for the congenital myasthenic syndromes: preparing for FAIR data in the genomic era. Orphanet J Rare Dis. 2018 Nov 26;13(1):211.
Vanhaesebrouck, A. E., et al. (2019). Brain 142(12): 3713-3727.
Webster RG, Cossins J, Lashley D, Maxwell S, Liu WW, Wickens JR, Martinez-Martinez P, de Baets M, Beeson D. Exp Neurol. 2013 Oct;248:286-98.

### DETAILS OF SEQUENCES

Any Sequence Listing filed with this patent application is fully incorporated herein as part of the description.
**SEQ ID NO: 1**
   **Human al CHRNA1 CDS sequence**
   CHRNA1-201 cds:protein_coding
**SEQ ID NO: 2**
   **Human α1 CHRNA1 amino acid sequence**
   CHRNA1-201 peptide: ENSP00000261007 pep:protein_coding
**SEQ ID NO: 3**
   **Human β1CHRNB1 CDS sequence**
   CHRNB1-201 cds:protein_coding
**SEQ ID NO: 4**
   **Human β1CHRNB1 amino acid sequence**
   >CHRNB1-201 peptide: ENSP00000304290 pep:protein_coding
**SEQ ID NO: 5**
   **Human δ CHRND CDS sequence**
   >CHRND-201 cds:protein_coding
**SEQ ID NO: 6**
   **Human δ CHRND amino acid sequence**
   >CHRND-201 peptide: ENSP00000258385 pep:protein_coding
**SEQ ID NO: 7**
   **Human ε CHRNE CDS sequence**
   >CHRNE-203 cds:protein_coding
**SEQ ID NO: 8**
   **Human ε CHRNE amino acid sequence**
   >CHRNE-203 peptide: ENSP00000497829 pep:protein_coding
**SEQ ID NO: 9**
   **Human γ CHRNG CDS sequence**
   >CHRNG-203 cds:protein_coding
**SEQ ID NO: 10**
   **Human γ CHRNG amino acid sequence**
   >CHRNG-203 peptide: ENSP00000498757 pep:protein_coding
**SEQ ID NO: 11**
   **Human *DOK7* CDS sequence**
   >DOK7-201 cds:protein_coding
**SEQ ID NO: 12**
   **Human Dok-7 polypeptide**
   >DOK7-201 peptide: ENSP00000344432 pep:protein_coding

## Claims

1. A *DOK7* gene or a Dok-7 polypeptide for use in preventing or treating a congenital myasthenic syndrome (CMS), wherein the CMS is:
(a) congenital myasthenic syndrome associated with AChR deficiency, or
(b) fast-channel congenital myasthenic syndrome (FCCMS).

2. The *DOK7* gene or a Dok-7 polypeptide for use as claimed in claim 1, wherein the CMS is due to a mutation in a gene encoding a nAChR subunit.

3. The *DOK7* gene or a Dok-7 polypeptide for use as claimed in claim 2, wherein the mutation is selected from the group consisting of:
(i) a mutation in the CHRNA1 gene which leads to AChR deficiency,
(ii) a mutation in the CHRNB1 gene which leads to AChR deficiency,
(iii) a mutation in the CHRND gene which leads to AChR deficiency,
(iv) a mutation in the CHRNE gene which leads to AChR deficiency,
(v) a mutation in the CHRNG gene which leads to AChR deficiency,
(vi) a mutation in the CHRNE gene which leads to FCCMS,
(vii) a mutation in the CHRNA1 gene which leads to FCCMS,
(viii) a mutation in the CHRNB1 gene which leads to FCCMS, and
(ix) a mutation in the CHRND gene which leads to FCCMS.

4. The *DOK7* gene or a Dok-7 polypeptide for use as claimed in claim 3, wherein the CMS disorder is due to a mutation in the CHRNE gene which leads to AChR deficiency, preferably wherein the mutation is a null mutation in the CHRNE gene.

5. The *DOK7* gene or a Dok-7 polypeptide for use as claimed in any one of claims 1-4, wherein the *DOK7* gene is present in a recombinant adeno-associated virus (rAAV) vector.

6. The *DOK7* gene or a Dok-7 polypeptide for use as claimed in claim 5, wherein the rAAV vector comprises a promoter operably-associated with the *DOK7* gene, preferably wherein the promoter is a CMV promoter, the human muscle creatine kinase promoter MHCK7, or a human skeletal actin (HSA) promoter.

7. The *DOK7* gene or a Dok-7 polypeptide for use as claimed in any one of claims 1-6, wherein the *DOK7* gene or a Dok-7 polypeptide is used or is administered in combination with one or more of:
(i) a cholinesterase inhibitor,
(ii) a beta-adrenergic receptor agonist,
(iii) an immuno-suppressor, or
(iv) a voltage-gated potassium channel blocker,
wherein the *DOK7* gene or Dok-7 polypeptide and the one or more of (i)-(iv) are administered simultaneously, separately or sequentially.

8. The *DOK7* gene or a Dok-7 polypeptide for use as claimed in claim 7, wherein:
(i) the cholinesterase inhibitor is pyridostigmine,
(ii) the beta-adrenergic receptor agonist is a beta2-adrenergic receptor agonist, preferably salbutamol,
(iii) the immuno-suppressor is prednisone, and/or
(iv) the voltage-gated potassium channel blocker is 3,4-DAP or 3,4-DAPP.

9. A pharmaceutical composition comprising a *DOK7* gene or Dok-7 polypeptide in combination with one or more of (i)-(iv):
(i) a cholinesterase inhibitor,
(ii) a beta-adrenergic receptor agonist,
(iii) an immuno-suppressor, or
(iv) a voltage-gated potassium channel blocker,
wherein the pharmaceutical combination is in the form of a combined preparation for simultaneous, separate or sequential use and wherein the pharmaceutical combination is for use in the treatment of a CMS, wherein the CMS is:
(a) congenital myasthenic syndrome associated with AChR deficiency, or
(b) fast-channel congenital myasthenic syndrome (FCCMS).

## Patentansprüche

1. *DOK7*-Gen oder Dok-7-Polypeptid zur Verwendung bei der Vorbeugung oder Behandlung eines kongenitalen Myasthenie-Syndroms (CMS), wobei das CMS Folgendes ist:
(a)Kongenitales Myasthenie-Syndrom in Verbindung mit einem AChR-Mangel, oder
(b)Fast-Channel-Kongenitales-Myasthenie-Syndrom (FCCMS).

2. *DOK7*-Gen oder Dok-7-Polypeptid zur Verwendung nach Anspruch 1, wobei das CMS auf eine Mutation in einem Gen zurückzuführen ist, das für eine nAChR-Untereinheit kodiert.

3. *DOK7*-Gen oder Dok-7-Polypeptid zur Verwendung nach Anspruch 2, wobei die Mutation ausgewählt ist aus der Gruppe bestehend aus:
(i) einer Mutation in dem CHRNA1-Gen, die zu AChR-Mangel führt,
(ii) einer Mutation in dem CHRNB1-Gen, die zu AChR-Mangel führt,
(iii) einer Mutation in dem CHRND-Gen, die zu AChR-Mangel führt,
(iv) einer Mutation in dem CHRNE-Gen, die zu AChR-Mangel führt,
(v) einer Mutation in dem CHRNG-Gen, die zu AChR-Mangel führt,
(vi) einer Mutation in dem CHRNE-Gen, die zu FCCMS führt,
(vii) einer Mutation in dem CHRNA1-Gen, die zu FCCMS führt,
(viii) einer Mutation in dem CHRNB1-Gen, die zu FCCMS führt, und
(ix) einer Mutation in dem CHRND-Gen, die zu FCCMS führt.

4. *DOK7*-Gen oder Dok-7-Polypeptid zur Verwendung nach Anspruch 3, wobei die CMS-Störung auf eine Mutation in dem CHRNE-Gen zurückzuführen ist, die zu einem AChR-Mangel führt, vorzugsweise wobei die Mutation eine Nullmutation in dem CHRNE-Gen ist.

5. *DOK7*-Gen oder Dok-7-Polypeptid zur Verwendung nach einem der Ansprüche 1-4, wobei das *DOK7*-Gen in einem rekombinanten Adeno-assoziierten Virus (rAAV)-Vektor vorhanden ist.

6. *DOK7*-Gen oder Dok-7-Polypeptid zur Verwendung nach Anspruch 5, wobei der rAAV-Vektor einen Promotor umfasst, der funktionsfähig mit dem *DOK7*-Gen assoziiert ist, vorzugsweise wobei der Promotor ein CMV-Promotor, der menschliche Muskelkreatinkinase-Promotor MHCK7 oder ein menschlicher Skelett-Actin (HSA)-Promotor ist.

7. *DOK7*-Gen oder Dok-7-Polypeptid zur Verwendung nach einem der Ansprüche 1-6, wobei das *DOK7*-Gen oder Dok-7-Polypeptid in Kombination mit einem oder mehreren der folgenden Elemente verwendet oder verabreicht wird:
(i) einem Cholinesterase-Inhibitor,
(ii) einem beta-adrenergen Rezeptor-Agonisten,
(iii) einem Immunsuppressivum, oder
(iv) einem spannungsabhängigen Kaliumkanalblocker,
wobei das *DOK7*-Gen oder Dok-7-Polypeptid und eine oder mehrere der folgenden von (i)-(iv) gleichzeitig, getrennt oder nacheinander verabreicht werden.

8. *DOK7*-Gen oder Dok-7-Polypeptid zur Verwendung nach Anspruch 7, wobei:
(i) der Cholinesterase-Inhibior Pyridostigmin ist,
(ii) der beta-adrenerge Rezeptor-Agonist ein beta2-adrenerger Rezeptor-Agonist ist, vorzugsweise Salbutamol,
(iii)das Immunsuppressivum Prednison ist, und/oder
(iv) der spannungsabhängige Kaliumkanalblocker 3,4-DAP oder 3,4-DAPP ist.

9. Pharmazeutische Zusammensetzung, umfassend ein *DOK7*-Gen oder Dok-7-Polypeptid in Kombination mit einem oder mehreren von (i)-(iv):
(i) einem Cholinesterase-Inhibitor,
(ii) einem beta-adrenergen Rezeptor-Agonisten,
(iii) einem Immunsuppressivum, oder
(iv) einem spannungsabhängigen Kaliumkanalblocker,
wobei die pharmazeutische Kombination in Form eines Kombinationspräparats zur gleichzeitigen, getrennten oder aufeinanderfolgenden Anwendung vorliegt und wobei die pharmazeutische Kombination zur Verwendung bei der Behandlung eines CMS bestimmt ist, wobei das CMS Folgendes ist:
(a) kongenitales Myasthenie-Syndrom in Verbindung mit einem AChR-Mangel, oder
(b) Fast-Channel-Kongenitales-Myasthenie-Syndrom (FCCMS).

## Revendications

1. Gène *DOK7* ou polypeptide Dok-7 pour une utilisation dans la prévention ou le traitement d'un syndrome myasthénique congénital (CMS), dans lequel le CMS est :
(a) le syndrome myasthénique congénital associé à un déficit en AChR, ou
(b) le syndrome myasthénique congénital à canal rapide (FCCMS).

2. Gène *DOK7* ou polypeptide Dok-7 pour une utilisation selon la revendication 1, dans lequel le CMS est dû à une mutation dans un gène codant une sous-unité nAChR.

3. Gène *DOK7* ou polypeptide Dok-7 pour une utilisation selon la revendication 2, dans lequel la mutation est sélectionnée parmi le groupe consistant en :
(i) une mutation du gène CHRNA1 qui conduit à un déficit en AChR,
(ii) une mutation du gène CHRNB1 qui conduit à un déficit en AChR,
(iii) une mutation du gène CHRND qui conduit à un déficit en AChR,
(iv) une mutation du gène CHRNE qui conduit à un déficit en AChR,
(v) une mutation du gène CHRNG qui conduit à un déficit en AChR,
(vi) une mutation du gène CHRNE qui conduit à un FCCMS,
(vii) une mutation du gène CHRNA1 qui conduit à un FCCMS,
(viii) une mutation du gène CHRNB1 qui conduit à un FCCMS, et
(ix) une mutation du gène CHRND qui conduit à un FCCMS.

4. Gène *DOK7* ou polypeptide Dok-7 pour une utilisation selon la revendication 3, dans lequel le trouble CMS est dû à une mutation du gène CHRNE qui conduit à un déficit en AChR, de préférence dans lequel la mutation est une mutation nulle du gène CHRNE.

5. Gène *DOK7* ou polypeptide Dok-7 pour une utilisation selon l'une quelconque des revendications 1 à 4, dans lequel le gène *DOK7* est présent dans un vecteur de virus adéno-associé recombinant (rAAV).

6. Gène *DOK7* ou polypeptide Dok-7 pour une utilisation selon la revendication 5, dans lequel le vecteur rAAV comprend un promoteur associé de manière fonctionnelle au gène *DOK7,* de préférence dans lequel le promoteur est un promoteur CMV, le promoteur de créatine kinase musculaire humaine MHCK7, ou un promoteur d'actine squelettique humaine (HSA).

7. Gène *DOK7* ou polypeptide Dok-7 pour une utilisation selon l'une quelconque des revendications 1 à 6, dans lequel le gène *DOK7* ou un polypeptide Dok-7 est utilisé ou administré en combinaison avec l'un ou plusieurs parmi :
(i) un inhibiteur de cholinestérase,
(ii) un agoniste des récepteurs bêta-adrénergiques,
(iii) un immunosuppresseur, ou
(iv) un bloqueur de canal potassique sensible au potentiel,
dans lequel le gène *DOK7* ou le polypeptide Dok-7 et l'un ou plusieurs parmi (i) à(iv) sont administrés simultanément, séparément ou en séquence.

8. Gène *DOK7* ou polypeptide Dok-7 pour une utilisation selon la revendication 7, dans lequel :
(i) l'inhibiteur de cholinestérase est la pyridostigmine,
(ii) l'agoniste des récepteurs bêta-adrénergiques est un agoniste des récepteurs bêta-2-adrénergiques, de préférence le salbutamol,
(iii) l'immunosuppresseur est la prednisone, et/ou
(iv) le bloqueur de canal potassique sensible au potentiel est la 3,4-DAP ou la 3,4-DAPP.

9. Composition pharmaceutique comprenant un gène *DOK7* ou un polypeptide Dok-7 en combinaison avec l'un ou plusieurs parmi (i) à (iv) :
(i) un inhibiteur de cholinestérase,
(ii) un agoniste des récepteurs bêta-adrénergiques,
(iii) un immunosuppresseur, ou
(iv) un bloqueur de canal potassique sensible au potentiel,
dans laquelle la combinaison pharmaceutique se présente sous la forme d'une préparation combinée pour une utilisation simultanée, séparée ou en séquence et dans laquelle la combinaison pharmaceutique est pour une utilisation dans le traitement d'un CMS, dans laquelle le CMS est :
(a) le syndrome myasthénique congénital associé à un déficit en AChR, ou
(b) le syndrome myasthénique congénital à canal rapide (FCCMS).
